# EUROPEAN PATENT SPECIFICATION

(11) **EP 0 938 304 B1**
(45) Date of publication and mention of the grant of the patent: **15.12.2004**
(21) Application number: 97941381.2
(22) Date of filing: 29.08.1997
(51) Int. Cl.: A61K 31/16, A61K 35/78, A61P 25/00, A61P 25/08, A61P 43/00, A61P 25/14

(54) **ISOVALERAMIDE FOR THE TREATMENT OF CONVULSIONS, EPILEPSY, HEADACHE AND SPASTICITY**
ISOVALERAMID ZUR BEHANDLUNG VON SCHÜTTELKRÄMPFEN, EPILEPSIE, KOPFSCHMERZEN UND SPASTISCHER LÄHMUNG
L'ISOVALERAMIDE DESTINEE AU TRAITEMENT DES CONVULSIONS, L'EPILEPSIE, LA CEPHALGIE ET LA SPASTICITE

(30) Priority: 30.08.1996 US 25050 P
(43) Date of publication of application: 01.09.1999
(73) Proprietor: NPS PHARMACEUTICALS, INC., Salt Lake City Utah 84108 (US)
(72) Inventor: ARTMAN, Linda, D., Salt Lake City, UT 84108 (US); BALANDRIN, Manuel, F., Sandy, UT 84093 (US)
(74) Representative: Maschio, Antonio, Dr.
(86) International application number: PCT/US1997/015272
(87) International publication number: WO 1998/008498

(56) References cited:
- WO-A-94/28888
- DE-A- 2 230 626
- US-A- 3 485 851
- KEANE ET AL.: "EFFECT OF VALPROATE ON BRAIN GABA : COMPARISON WITH VARIOUS MEDIUM CHAIN FATTY ACIDS" PHARMACOL. RES. COMMUN., vol. 17, no. 6, 1985, pages 547-555, XP002049841
- HAZELHOFF ET AL.: "Antispasmodic effects of Valeriana Compounds: An in-Vivo and in-Vitro Study on the Guinea-Pig Ileum" ARCH. INT. PHARMACODYN., vol. 257, no. 2, 1982, pages 274-287, XP002049842
- MORAZZONI ET AL.: "Valeriana officinalis: traditional use and recent evaluation of activity" FITOTERAPIA, vol. 66, no. 2, 1995, pages 99-112, XP002049843
- BALANSARD ET AL.: "Critères de choix d'un extrait de Viburnum, Viburnum Prunifolium L., en fonction de ses actions toniveineuse et spasmolytique." PLANT MED. PHYTOTHER., vol. 17, no. 3, 1983, pages 123-132, XP002049844
- FURNADJIEV G.: "PHYTOTHERAPEUTIC AND EXPERIMENTAL DATA ON SOME MEDICINAL PLANTS WITH SPASMOLYTIC EFFECT AND THEIR APPLICATION IN GASTROINTESTINAL DISEASES" PROBL. VATR. MED., vol. 8, no. 3, 1980, pages 79-86, XP002049845
- WAGNER ET AL.: "Ueber die spasmolytische Wirkung des Badrians" PLANTA MED., vol. 37, no. 1, 1979, pages 84-86, XP002049846
- TAILLANDIER ET AL.: "Recherches dans la série dipropylacétique XII. Acides et alcools aliphatiques ramifiés anticonvulsivants" EUR. J. MED. CHEM., vol. 10, no. 5, 1975, P0C038, pages 453-462, XP002049847
- WEISS R.F.: "Herbal Medicine" 1988 , AB ARCANUM , GOTHENBURG, SWEDEN XP002049848 00014 Chapter 11, pages 280-286, 290 and chapter 12 page 307, column 1.
- B ZANGER-BEAUQUESNE ET AL.: "Plantes médicinales des régions tempérées" 1980 , MALOINE S.A. , PARIS XP002049849 00002 see pages 78, 363, 364 and 365

## Description

### BACKGROUND

The present invention relates to the use of isovaleramide in the preparation of compositions for treating pathological conditions, such as spasticity and convulsions, the symptoms of which are alleviated by a mild depression of activity in the central nervous system (CNS), without producing undesirable excessive sedation or muscle weakness in animal subjects, including humans.

Many agents currently employed in the treatment of pathologies such as spasticity and convulsions display troubling side-effect profiles which limit their longterm clinical utility. Among these agents, for example, are the benzodiazepines, which can cause cognitive blunting. Two other agents are valproate, which exhibits hepatotoxicity, and baclofen, which can produce excessive muscle weakness and sedation, limiting the therapeutic potential for both drugs.

### SUMMARY

Accordingly, it is an object of the present invention to provide a therapeutic approach for the treatment of various pathologies by effecting a mild depression in CNS activity without producing excessive sedation, muscle weakness, fatigue, or hepatotoxicity.

It is another object, of the present invention to provide compositions for use in a novel anticonvulsant therapy.

In accomplishing these and other objectives, there has been provided, according to one aspect of the present invention, a use of isovaleramide in the preparation of a pharmaceutical formulation for use in a method of treating a pathology selected from the group consisting of convulsions, epilepsy, headache and spasticity, whereby at least one symptom of said pathology is alleviated.

Other objects, features, and advantages of the present invention will become apparent from the following detailed description.

### BRIEF DESCRIPTION OF THE DRAWINGS

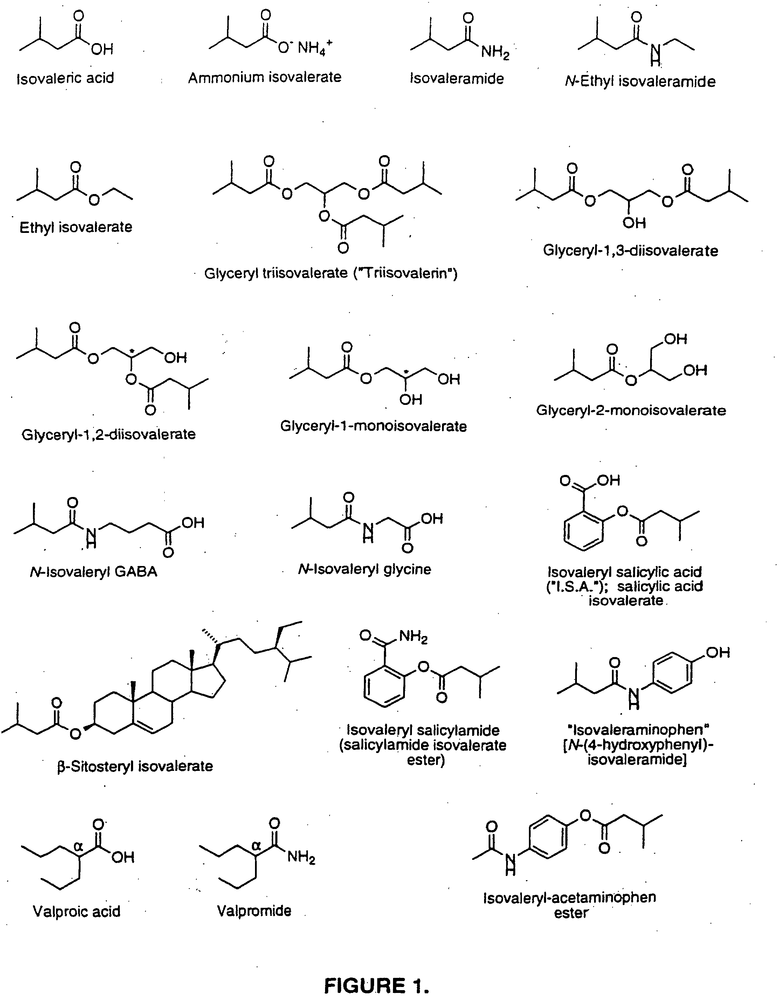

Figure 1 depicts the structures of various compounds, including isovaleramide.

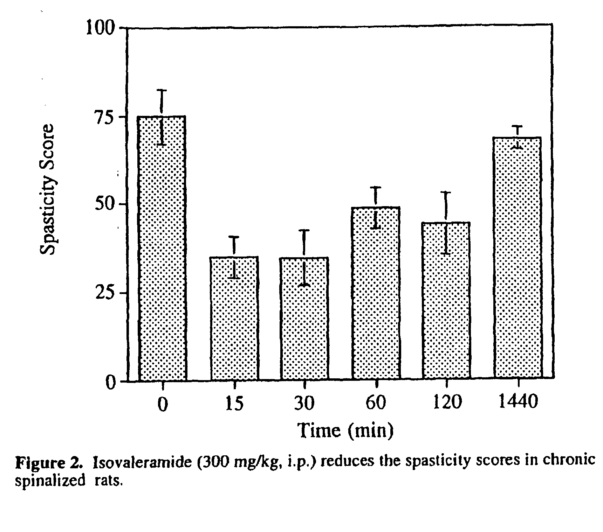

Figure 2 portrays the effect of isovaleramide (at 300 mg/kg, i.p.) on gross observational spasticity scores elicited by a metal probe applied to the abdomen in the chronic spinalized rat. Each rat served as its own control; there were three rats per group. The bar at time zero represents pre-treatment control values.

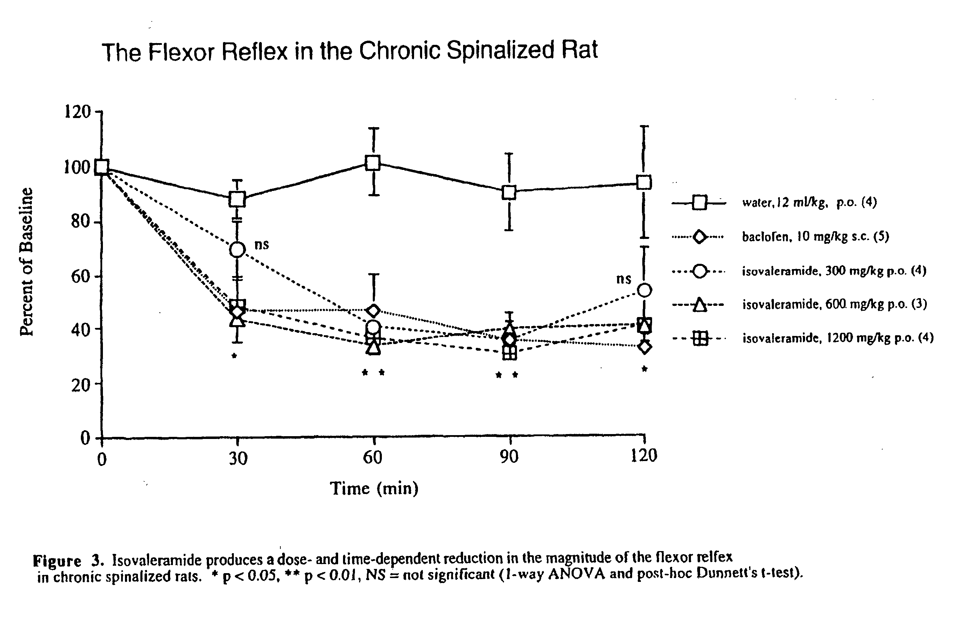

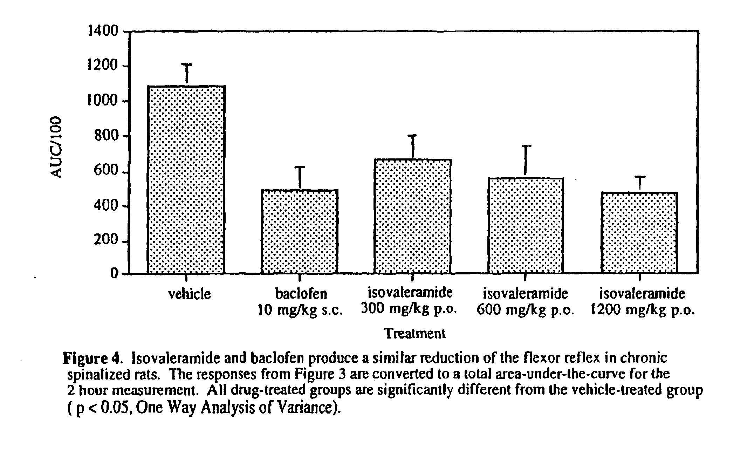

Figure 3 illustrates a dose- and time-dependent reduction of the flexor reflex, an electrophysiological measure of spasticity, in the chronic spinalized rat. The effects of isovaleramide (300, 600, and 1200 mg/kg p.o.), baclofen (10 mg/kg s.c.), and vehicle (water, 12 ml/kg p.o.) are shown at pre-treatment (time zero) and at 30, 60, 90, and 120 minutes post-administration. At all doses, isovaleramide caused a significant decrease in the magnitude of the flexor reflex, comparable to that observed with baclofen. statistical significance was assessed by one-way analysis of variance (ANOVA) and post-hoc Dunnett's t-test: p < 0.05 (*); p < 0.01 (**); NS = not significant.
Figure 4 shows that isovaleramide and baclofen, a known antispasticity agent, produced a similar reduction of the flexor reflex in the chronic spinalized rat. The responses from Figure 3 were converted to a total-area-under-the-curve for the two-hour measurement. All drug-related groups were significantly different from the vehicle (p < 0.05, ANOVA).

### DETAILED DESCRIPTION OF THE PREFERRED EMBODIMENTS

### 1. OVERVIEW

The inventors have discovered that isovaleramide, can be administered *in vivo* to effect a mild depression of CNS activity. That is, these agents depress CNS activity, by enhancing inhibitory (or decreasing excitatory) neurotransmission centrally, without complete suppression of all activity. Pursuant to the present invention, therefore, a subject who receives such an agent is not overtly sedated, anesthetized, or paralyzed in the context, for example, of decreasing seizures (no anesthesia), decreasing muscle tone (no paralysis), eliciting a calmative effect (no sedation), or ameliorating an ambulatory syndrome such as spasticity (no weakness or flaccidity).

A number of pathologies, exemplified by headaches (chronic, cluster, migraine), spasticity, convulsions and epilepsy, have at least one symptom that is alleviated by a mild CNS depression. Accordingly, an individual who suffers from such a pathology is a candidate for therapy that entails, pursuant to the present invention, the individual's receiving a pharmaceutical formulation of isovaleramide.

It is believed that the compounds of the present invention act via a GABAergic mechanism and, hence, bear a pharmacological similarity to known drugs which are considered to enhance central GABAergic neurotransmission. Like many of the extant drugs, such as the barbiturates, the benzodiazepines, gabapentin, valproate, vigabatrin, and progabide, the compounds of the present invention are effective in treating pathological conditions, illustrated by those mentioned above, that are thought to arise from a defect in the regulation of inhibitory (GABA- and/or glycine-related) neurotransmission. This regulation may occur by a direct or modulatory effect at CNS receptors or by impact on a metabolic pathway which heightens GABA or glycine levels and/or which reduces levels of an excitatory neurotransmitter like glutamate. See Ruggero *et al*., *in* ANTIEPILEPTIC DRUGS (4th ed.), pages 581-88 (Raven Press 1995); Nogrady, MEDICINAL CHEMISTRY: A BIOCHEMICAL APPROACH (2d ed.), pages 225-39 (Oxford University Press 1988); Fonnum and Morselli, respectively, in PSYCHOPHARMACOLOGY: THE THIRD GENERATION OF PROGRESS, pages 173-82 and 183-95 (Raven Press 1987). Despite a pharmacological analogy to the known drugs mentioned above, however, the present invention surprisingly does not engender the disadvantageous side effects associated with conventional drug therapies in this area, such as the hepatotoxicity that arises with valproate administration.

### 2. EXEMPLARY PATHOLOGIES AMELIORATED BY A MILD DEPRESSION OF CNS ACTIVITY

***SPASTICITY*:** "Spasticity is frequently defined as an upper [*i*.*e*., CNS] motor neuron disorder characterized by a velocity dependent increase in tonic stretch reflexes (muscle tone) with exaggerated tendon jerks resulting from hyperexcitability of the stretch reflex." Lance, Symposia synopsis *in* SPASTICITY - DISORDERED MOTOR CONTROL, Feldman *et al*. (eds.) (1980). An increase in tonic stretch reflexes, however, is only one of the many symptoms present in a disordered motor function caused by an upper neuron lesion in a variety of neurological disorders; thus, such a disordered motor function is variable in its etiology and presentation.

Major disease states and conditions associated with spasticity include multiple sclerosis, cerebral palsy, stroke, trauma or injury to the spinal cord, and closed head trauma. There are "positive symptoms" that can occur with spasticity, such as the Babinski response, painful flexor or extensor spasms, increased or exaggerated deep tendon reflexes, and clonus. Other symptoms, referred to as "negative symptoms," include weakness, fatigue, lack of dexterity, and paralysis. It is the combination of these positive and negative signs and symptoms that is denoted clinically as "spastic paresis" (spastic paralysis). Pain, impairment of sleep, and various degrees of loss of general motor function are also associated with spasticity.

The pathological states observed in spasticity are fundamentally different at the physiological level from the commonly experienced acute muscular aches, strains, and sprains that occur from a localized external insult to a particular muscle, *i*.*e*., outside of or peripheral to the CNS. These pathological states also are different from the relatively common involuntary spasms of smooth muscle, such as vascular spasms, bladder spasms, and bronchial spasms. Such non-spastic (non-CNS), peripheral or localized symptoms are commonly treated with so-called "antispasmodic" or "spasmolytic" agents, but these generally are not useful in treating spasticity. Cedarbaum & Schleifer, "Drugs for Parkinson's Disease, Spasticity and Acute Muscle Spasms," *in* GOODMAN AND GILMAN'S THE PHARMACOLOGICAL BASIS OF THERAPEUTICS, 8th ed. [hereafter GOODMAN AND GILMAN'S], pages 463-484 (Pergamon Press 1990).

The pharmaceutical formulations employed in accordance with the present invention can effect a centrally mediated decrease in muscle tone and, hence, are useful for the acute or chronic alleviation of one or more symptoms of spasticity. In this context, "spasticity" refers to a heightened tone of skeletal muscle which is manifested by symptoms such as (but not limited to) painful flexor or extensor spasms, increased or exaggerated deep tendon reflexes, hyperreflexia, loss of dexterity, muscular weakness, exaggerated tendon jerks, and clonus. The phrase "antispasticity agent" refers here to a composition that is useful for the symptomatic treatment of spasticity, as demonstrated by the alleviation of at least one of the following manifestations of spasticity: painful flexor or extensor spasms, increased or exaggerated deep tendon reflexes, hyperreflexia, loss of dexterity, muscular weakness, exaggerated tendon jerks, and clonus. Accordingly, the "alleviation" of spasticity refers here to the lessening of one or more symptoms of spasticity, including but not limited to painful flexor or extensor spasms, increased or exaggerated deep tendon reflexes, hyperreflexia, loss of dexterity, muscle weakness, exaggerated tendon jerks, and clonus.

Spasticity is associated with multiple sclerosis, stroke, head trauma, spinal cord injuries, cerebral palsy, and other neurodegenerative diseases, disorders, and conditions. Spasticity is distinct from acute muscle spasms, which may be associated with a variety of conditions different from those leading to spasticity. These acute muscle spasm-causing conditions include trauma, inflammation, anxiety, and/or pain.

The difference between spasticity and acute muscle spasms is illustrated by the fact that agents useful for the treatment of muscle spasms are not useful for treating spasticity associated with chronic neurological diseases. Cedarbaum & Schleifer (1990), *supra.* Likewise, agents used heretofore to treat spasticity associated with chronic neurological disorders have not been employed in treating acute muscle spasms, except for the benzodiazepines, such as diazepam (Valium®), which are recognized also to have muscle-relaxant activity as well as anxiolytic and analgesic properties. By contrast, the present invention achieves a centrally mediated decrease in muscle tone which, in turn, addresses the particular symptoms of spasticity.

***CONVULSIVE DISORDERS*:** Due to the widespread availability of reasonably predictive and experimentally accessible animal models of convulsant states, a number of clinically useful anticonvulsants have been prepared and developed. For example, see Cereghino *et al*., "Introduction," *in* ANTIEPILEPTIC DRUGS, 4th ed., pages 1-11 (Raven Press 1995). "In many patients, seizures can be controlled with currently available antiepileptic drugs, but 25 to 30 percent of patients continue to have seizures despite optimal therapy, while many others experience unacceptable side effects." Dichter *et al*., *Drug Therapy* 334: 1583 (1996).

Thus, many anticonvulsants in clinical use are plagued by the occurrence of significant side effects, including troublesome daytime sedation, muscular weakness, tolerance, gingival hyperplasia, blood dyscrasias, and potentially fatal hepatotoxicity. Many of these side effects are especially of concern in the clinical management (treatment) of epilepsy in children.

The present invention can be used to treat convulsive disorders such as epilepsy. That is, the pharmaceutical compositions of the invention display "anticonvulsant activity," which is evidenced by a reduction of the severity, number, or duration of convulsions in animal models of epilepsy. Accordingly, the inventive pharmaceutical compositions should be useful in treating conditions such as but not limited to simple partial seizures, complex partial seizures, status epilepticus, and trauma-induced seizures, as occur following head injury or surgery.

Epilepsy is a common disorder which has many causes, and it can be very difficult to control, often requiring treatment for many years to keep seizures under control. "At this time, there is no satisfactory treatment for epilepsy in a substantial proportion of patients. Clinical trials have shown that certain patients have a better response to one drug than another, even when the patients have similar types of seizures and the drugs have similar mechanisms of action. The frequency and severity of side effects also varies substantially. Thus, multiple medications with different mechanisms of action and attendant side effects will be needed for treatment of epilepsy until either epilepsy can be cured or a potent, safe new drug with broad activity is discovered" and developed. Dichter *et al*. (1996), *supra*.

***HEADACHES*:** Headaches of the migraine type (Hering & Kuritzky, *Cephalalgia* 12: 81-84 (1992)), the cluster type (Hering & Kuritzky, *loc. cit*. 9: 195-98 (1989)) and the chronic type (Mathew & Sabiha, *Headache* 31: 71-74 (1991)) have been treated by the administration of valproate. Interactions with the GABAergic system are thought to play a major role in the etiology of these headaches and in the associated valproate therapy. For this reason, the present invention can alleviate symptoms associated with each of the three headache types, without the adverse side effects of valproate therapy.

### 3. METHODS FOR PREPARING PHARMACEUTICAL FORMULATIONS

The rhizomes and roots of *Valeriana* spp. (common name: valerian; family Valerianaceae) have been used for medicinal purposes since ancient times. The most commonly used valerian preparations include aqueous and hydroalcoholic extracts, such as tinctures, intended for oral administration. In addition, ammoniated valerian tinctures were used medicinally in the English-speaking world since at least the beginning of the seventeenth century. Hobbs, *HerbalGram* No. 21: 19-34 (1989). In the last three decades, the sedative and antispasmodic properties of valerian preparations have been attributed primarily to the presence of chemically labile monoterpenoid iridoid triester compounds called valepotriates ("valerian-epoxy-triesters (-ates)").

Isovaleramide has been isolated from valerian plants, most probably as an isolation artifact following treatment with ammonia. Buckova *et al*., *Cesk*. *Farm. 26:* 308 (1977); *Chem. Abstr*. *88:* 86063z (1978); see also Bos *et al*. and Fuzzati *et al*., *Phytochem*. *Anal.* 7: 143, 76 (1996). More Recently, isovaleramide was shown to exhibit low acute toxicity *in vivo*, no mutagenic potential, and clinically useful anxiolytic properties. U.S. patent No. 5,506,268; PCT application WO 94/28,888. Methods for preparing isovaleramide are well known. only slightly volatile, thereby minimizing any unpleasant odors. Furthermore, in pure form these esters possess the advantage of having neutral to pleasant odors, in contrast to the extremely unpleasant odors of isovaleric acid and its salts, such as the ammonium, sodium, potassium, and zinc isovalerate salts. Moreover, whereas ethyl isovalerate is a liquid, the glyceryl mono-, di-, and tri-isovalerates, isovaleryl salicylate, and β-sitosteryl isovalerate are expected to be solids at room temperature, thereby facilitating their formulation into various standard solid and liquid oral dosage forms well known in the art, such as tablets (*e*.*g*., uncoated tablets, enteric-coated tablets, and film-coated tablets), capsules, gelcaps, powders, concentrates (drops), elixirs, tinctures, and syrups.

In addition isovaleramide, can be prepared by methods well known in the art. See, for example, March, ADVANCED ORGANIC CHEMISTRY: REACTIONS, MECHANISMS, AND STRUCTURE, 4th ed. (John Wiley and Sons 1992).

The pharmaceutical formulations of the present invention can be prepared according to known methods to prepare pharmaceutically useful compositions, whereby active agents are combined in a mixture with a pharmaceutically acceptable carrier. For instance, see REMINGTON'S PHARMACEUTICAL SCIENCES and GOODMAN AND GILMAN'S, both cited above. A composition is said to be in a "pharmaceutically acceptable carrier" if its administration can be tolerated by a recipient patient. Sterile phosphate-buffered saline is one example of a pharmaceutically acceptable carrier. Other suitable carriers (e.g. saline and Ringer's solutions) are well known to those skilled in the art. See, for example, REMINGTON'S PHARMACEUTICAL SCIENCES, *supra.*

In general, the dosages of the antispasticity and anticonvulsant agents described herein will vary depending upon such factors as the patient's age, weight, height, sex, general medical condition, and previous medical history. For purposes of therapy, a compound of the present invention and a pharmaceutically acceptable carrier are administered to a subject in need of such treatment in a therapeutically effective amount. The combination of active agent and carrier is said to be administered in a "therapeutically effective amount" if the amount administered is physiologically significant. An agent is physiologically significant if its presence results in a detectable change in the physiology of a recipient patient. In the present context, for example, an antispasticity agent is physiologically significant if the presence of the agent results in the alleviation of spasticity, while an anticonvulsant, agent is physiologically significant if the presence of the agent results in the reduction of the severity, number, or duration of convulsions.

Isovaleramide can be administered orally using solid oral dosage forms such as enteric-coated tablets, caplets, gelcaps, or capsules, or via liquid oral dosage forms such as syrups or elixirs. The indicated dosage of isovaleramide and related compounds as antispasticity agents is on the order of 100-1000 mg per dose, and preferably, 300-600 mg per dose. Unit solid oral dosage forms preferably contain about 200-350 mg per tablet or capsule, which typically would be taken 1-2 at a time for a maximum of four times per day, at a dosage of 1-20 mg/kg body weight. Liquid formulations can also be employed with active ingredient compositions so as to provide 1-2 teaspoonfuls per dose. Furthermore, corresponding reduced dosage pediatric chewable and liquid oral dosage forms can also be administered. These compounds also can be added to foods and beverages in the form of drops (with a dropper from a "concentrate" preparation) for oral administration. In addition, compounds such as isovaleramide may be formulated into chewing gum to facilitate oral delivery and absorption.

Alternatively, isovaleramide can be administered by injection or other systemic routes, such as transdermal or transmucosal administration, for example, nasally, bucally, or rectally, via suppositories. oral administration is much more convenient, however, and therefore is preferred.

For use in an oral anticonvulsant composition, the dosage level of active ingredient(s) is on the order of 100-1000 mg per dose, and preferably, 200-600 mg per dose, or 1-20 mg/kg body weight.

In addition to a use in humans, isovaleramide can be used, for example, as antispasticity agents or anticonvulsant agents, in animals such as cats, dogs, birds, horses, cattle, mink, poultry, and fish. In such cases the active compound may be administered by injection or other systemic routes, such as transdermal or transmucosal administration (for example, rectal administration via suppositories), or orally by addition to food or drink. As an antispasticity agent, the indicated oral dosage of isovaleramide and/or related compounds per kilogram of body weight of such animals is about 1-1000 mg/kg, depending upon the species of animal and the route of administration. A preferred range for oral dosage is about 200-600 mg/kg body weight.

The indicated oral dosage of isovaleramide per kilogram body weight as anticonvulsant agents for animals is in the range of about 1-1000 mg/kg, depending upon the species of animal and the route of administration. A preferred range for oral dosage is about 100-600 mg/kg body weight.

The present invention thus contemplates a variety of pharmaceutical compositions containing isovaleramide, as active ingredients that are suitable for oral, parenteral, transdermal, transmucosal, intranasal, buccal, or rectal administration. Although such compounds may be present as incidental by-products in certain pharmaceutical formulations which are outside the scope of the present invention, the common feature of the present formulations is that isovaleramide, isovaleric acid, and/or its pharmaceutically acceptable salts, substituted amides, and alcohol esters is present in a standardized amount. That is, the pharmaceutical formulations contain a predetermined, chemically defined, and quantifiable amount of at least one of such compounds to enable the determination of the quantity of a particular composition required to achieve the dosage levels described herein.

It is further understood that isovaleramide can be used in combination with other pharmaceutically active ingredients.

### 4. DEMONSTRATING THERAPY-IMPLICATING ACTIVITY

The suitability and effectiveness of a given pharmaceutical formulation for the alleviation of a pathology, as discussed above, can be demonstrated using animal models such as (but not limited to) those described below.

### (a) The Mutant Spastic Mouse

The mutant spastic mouse is a homozygous mouse that carries an autosomal recessive trait of genetic spasticity. The mouse is normal at birth, and then the mouse develops a coarse tremor, abnormal gait, skeletal muscle rigidity, and abnormal righting reflexes at two to three weeks of age. No structural abnormalities have been found. Rather, the mouse has a deficit of glycine receptors throughout the central nervous system. Drugs that either potentiate the binding or synthesis of GABA, such as valproate and the benzodiazepines, are effective compounds to ameliorate some of the symptoms of spasticity in this model, as well as in humans.

The assessment of spasticity in the mutant spastic mouse can be performed by electrophysiological assessment similar to the EMG recordings described below. One can also, on a more crude scale, measure righting. These mice have an abnormal delayed righting reflex when placed on their backs. Any righting reflex over one second is considered abnormal. Most normal mice cannot even be placed on their backs. Tremor can be evaluated by holding mice by their tails and rating the tremor subjectively by "absent," "slight," "moderate," or "severe." Flexibility is assessed by placing the mouse on a glass object with smoothly rounded grooves and rims. The glass object is lifted about 12 inches above the table and slowly tilted until almost vertical. Normal mice will climb about the object for a minute or more before falling to their feet. Spastic mice usually remain stiffly in one position and soon fall onto their backs. Chai *et al*., *Proc. Soc. Exptl*. *Biol. Med*. 109: 491 (1962).

### (b) The Acute/Chronic Spinally Transected Rat And The Acute Decerebrate Rat

There are several models of spasticity including the acute decerebrate rat, the acute or chronic spinally transected rat, and the chronically spinal cord-lesioned rat. The acute models, although of proven value in elucidating the mechanisms involved in the development of spasticity, have come under criticism due to the fact that they are acute. The animals usually die or have total recovery from spasticity. The spasticity develops immediately upon intervention, unlike the spasticity that evolves in the human condition of spasticity, which most often initially manifests itself as a flaccid paralysis. Only after weeks and months does spasticity develop in humans. Some of the more chronic-lesioned or spinally transected models of spasticity do post-operatively show flaccid paralysis. At approximately four weeks post-lesion/transection, the flaccidity changes to spasticity of variable severity. Although all of these models have their own particular disadvantages and lack of true representation of the human spastic condition, they have provided much information about the nature of spasticity. These models have also provided methods to test various treatment paradigms that have led to similar treatments being tested in humans. Many of these models have also made use of different species, such as cats, dogs, and primates. Baclofen, diazepam, and tizanidine are effective on different parameters of spasticity (EMG recordings, H-reflex, the H/M ratio, mono- and polysynaptic reflexes, clonus, hyperreflexia) in these models.

### (c) Primary Observation Irwin Test In The Rat

This method is based on that described by Irwin, *Psychopharmacologia* 13: 222-57 (1968). It is used to detect physiological, behavioral, and toxic effects of a test substance, and indicates a range of dosages that can be used for later experiments. Typically, rats (three per group) are administered the test substance and are then observed in comparison with a control group given vehicle. Behavioral modifications, symptoms of neurotoxicity, pupil diameter, and rectal temperature are recorded according to a standardized observation grid derived from that of Irwin. The grid contains the following items: mortality, sedation, excitation, aggressiveness, Straub tail, writhes, convulsions, tremor, exophthalmos, salivation, lacrimation, piloerection, defecation, fear, traction, reactivity to touch, loss of righting reflexes, sleep, motor incoordination, muscle tone, stereotypies, head-weaving, catalepsy, grasping, ptosis, respiration, corneal reflex, analgesia, abnormal gait, forepaw treading, loss of balance, head twitches, rectal temperature, and pupil diameter. Observations are performed at 15, 30, 60, 120, and 180 minutes following administration of the test substance, and also 24 hours later. The test substance is usually administered intraperitoneally (i.p.).

### (d) Rotarod Test In The Rat and Mouse

This is a test of neurological deficits using the method described by Dunham *et al*., *J. Am. Pharm*. *Assoc.* 46: 208-09 (1957). Rats or mice are placed on a rod rotating at a speed of eight turns per minute. The number of animals which drop off the rod before three minutes is counted and the drop-off times are recorded (maximum: 180 sec). Ten rats are studied per group and the test is performed blind. The test compound is administered i.p. 60 min prior to testing. Diazepam, a benzodiazepine, is administered at 8 mg/kg, i.p., as the reference substance. A control group administered the vehicle is also included in the study.

### (e) Anticonvulsant Activity

There are numerous *in vivo* models involving different kinds of seizures and behavioral effects that are relevant for clinically distinct forms of epilepsy. It therefore is prudent to test for effects in several models, because it may be an oversimplification to suppose that the same mechanism underlies all forms of seizure activity.

One useful model is provided by the Frings audiogenic seizure-susceptible mouse, a model of reflex epilepsy. At the time of testing, individual mice are placed into a round Plexiglas chamber and exposed to a sound stimulus of 110 decibels, 11 kHz, for 20 seconds. Animals not displaying tonic hindlimb extensions were considered protected. In addition, the seizure score for each mouse can be recorded as: (1) running for less than 10 seconds; (2) running for greater than 10 seconds; (3) clonic activity of limbs and/or vibrissae; (4) forelimb extension/hindlimb flexion; and (5) hindlimb extension.

The average seizure score can be calculated for each group of mice employed in the dose-response study. At each dose, mice are also tested on a rotarod for testing of motor impairment (toxicity). Testing for motor impairment on the rotarod involves placing a mouse for a three-minute trial period on a one-inch diameter rod rotating at six revolutions per minute. If the mouse falls off of the rotating rod three times within the three-minute time period, it is considered a toxic response.

### (f) Anti-Manic Activity

To assess the possible use of compounds in the treatment of affective mood disorders, one can employ the amphetamine-induced hyperactivity model in rats. In addition to being a test for classical and atypical antipsychotic activity, this procedure has also been proposed as a simple animal model of manic behavior. Costall *et al*., *Brain Res.* 123: 89-111 (1977).

### (g) Neurogenic Inflammation Of The Meninges

Neurogenic inflammation within the meninges has been proposed as an event in the underlying pathology of migraine headaches. Lee *et al*., *Brit*. *J*. *Pharmacol*. 116: 1661-67 (1995). Compounds are tested for their ability to block the leakage of radiolabeled bovine serum albumin within the dura mater post trigeminal stimulation.

### (h) Analgesic Properties

There are many whole-animal assays for determining analgesic properties, such as writhing, hotplate, tail flick, arthritic pain, paw pressure tests, and the Bennet or Chung models of neuropathic pain. Albe-Fessard *et al*., in 13 ADVANCES IN PAIN RESEARCH AND THERAPY, pages 11-27 (Raven Press 1990).

### (i) Therapeutic Benefit Relative To Movement Disorders and Restlessness syndromes

Animal models exist for the study of movement disorders and restlessness syndromes, for example, drug-induced akathisias, serotonin syndrome, rotation induced by unilateral nigral lesions. Lloyd & Morselli (1987), *supra.* Additionally, individual case reports of anecdotal efficacy of compounds in humans have been a source for support for these indications. Mellick & Mellick (1995), *supra;* Olson *et al*., *Am. J. Med*. 102: 60-66 (1997).

The therapeutic effects of isovaleramide in various of the assays described above, combined with a general lack of toxicity, make the compounds of the present invention ideal agents for the treatment of the pathologies described above, including spasticity and convulsions/seizures. With this background, the present invention will be understood more readily by reference to the following examples.

### Example 1

### Isovaleramide Antispasticity Tests

### (1) Assessment of Spasticity in Chronic Spinally Transected Rats

In these studies, male albino Holtzman-derived rats (Harlan Sprague-Dawley Laboratories) weighing 270-530 grams were used as subjects. The animals were housed independently and had continuous access to food and water throughout the experiments. All procedures were reviewed and approved by the Institutional Animal Care and Use Committee. Animals were anesthetized using a mixture of isoflurane and oxygen at a flow rate of 4 liters/minute. The rats were then placed in a stereotaxic frame and anesthesia was maintained. An incision was made so that the paraspinal muscles could be retracted and a laminectomy performed between T6-T9. A one- to two-millimeter portion of the spinal cord was removed by evacuation and replaced with Gel foam to reduce bleeding, after which the incision was closed in layers.

Following the transection, rats were placed in a room in which the ambient temperature was raised to about 80°F with a space heater to maintain body temperature. On the following morning post-surgery, the hindquarters of the spinalized rats were bathed and their urine expressed manually by applying pressure to their bladders. Experiments were conducted between 21 and 28 days after surgery. For the first two weeks, these rats were given 0.25 ml of the antibiotic Sulfatrim Pediatric Suspension orally to prevent bladder infection. A commercial antibiotic cream was applied to any part of the skin that showed signs of decubitus lesions. Within approximately two weeks, all animals regained bladder control and were no longer given antibiotic treatment. Advokat, *Brain* *Res. 684:* 8 (1995). Assessment of spasticity was performed before and after drug treatment such that each animal served as its own control.

Initial assessment of spasticity was performed by the subjective scoring method of rating the resulting spasticity response elicited with an innocuous stimulus, *i.e.*, a metal probe, that was pressed against the lower abdomen at four specific sites. The spastic reaction was evaluated for each of the four trials using a scale ranging from zero (no spastic response in all four trials) to four (a maximum, tonic-clonic reaction elicited in all four trials). All spasticity scores, pre- and post-treatment, were transformed to indicate the percent spasticity such that a score of 0/4 = 0%, 1/4 = 25%, etc. These raw or normalized scores were analyzed with a one-way repeated measures ANOVA.

As shown in Figure 2, isovaleramide at a dose of 300 mg/kg, i.p., was efficacious at 15, 30, 60, and 120 minutes post-administration in reducing the spasticity scores (45-65%). By the next day, *i.e.*, by 1440 minutes (24 hours), the spasticity scores had essentially returned to baseline values. No overt behavioral toxicity or motor impairment was observed at this dose. The rats were alert and able to grasp with their non-paralyzed front paws as were the control, untreated rats.

With reference to Figure 3, the polysynaptic flexor-reflex responses, to test stimuli which activate high-threshold afferents, were recorded as EMG activity from the ipsilateral hamstring muscle. Supramaximal electric shocks were applied to the hindpaw, and recording electrodes were placed in the biceps femoris semitendinosus muscle. Five sets of stimuli were made at each time point. The flexor reflex was recorded, in both the pre-drug and the post-drug periods, every 30 minutes once a stable baseline response was achieved. See Hao *et al*., *Eur*. *J*. *Pharmacol.* 191: 407 (1990).

Thus, the responses were determined in spinalized rats by observing the flexor-reflex response (Figure 3) before treatment and at each of 30, 60, 90, and 120 minutes following administration of isovaleramide (300, 600, and 1200 mg/kg p.o.), baclofen (10 mg/kg s.c.) and vehicle (water, 12 ml/kg p.o.), respectively.

Isovaleramide at all doses was shown to reduce the magnitude of the flexor-reflex responses, at all time points in a chronic spinalized rat, with spasticity. In this model, no change in the H/M reflex was observed with either baclofen or isovaleramide.

In Figure 4, the responses from Figure 3 are converted to a total-area-under-the-curve format, covering the entire, two-hour measurement period. All drug-treated groups differed significantly from vehicle (p < 0.05), based on a one-way analysis of variance. Between the drug-treated groups, no differences were found in total reduction of the flexor reflex over the two-hour period (pairwise multiple comparison, Student-Newman-Keuls method).

### (2) Primary Observation Irwin Test in the Rat

Administered i.p. in the rat, isovaleramide induced no changes from saline-injected controls at doses up to 256 mg/kg. At 512 mg/kg, slight sedation from 60 to 120 minutes, loss of traction (observed in only one of three rats) at 120 minutes, and decreased muscle tone from 60 to 120 minutes were observed. At 1024 mg/kg, marked sedation up to 30 minutes was observed, becoming moderate up to 120 minutes, then slight at 180 minutes. Decreased fear was also observed at this dose up to 30 minutes and in one of three rats up to 120 minutes. Decreased reactivity to touch up to 120 minutes, decreased muscle tone up to 180 minutes, slight hypothermia up to 120 minutes, and an abnormal gait (rolling) from 60 to 80 minutes were also observed at this dose. Loss of grasping and loss of righting reflex occurred, in one of three rats, at 15 minutes at this dose.

### (3) Rotarod Test in the Rat and the Frings Mouse

Isovaleramide, administered at doses of 128, 256, and 512 mq/kg (i.p.) 60 minutes before a test on the rotarod, did not significantly affect rotarod performance in the rat. See Table 1. In contrast, diazepam dose-dependently decreased rotarod performance.

**Table 1**

| **Effects of Isovaleramide and Diazepam in the Rotarod Test in the Rat** | | | | |
|---|---|---|---|---|
| Dose of: Isovaleramide (mg/kg)^{a} | Number^{b} of Rats Falling | Drop-Off Time (sec) | | |
| | | Mean ± S.E.M. | t value | % change from control |
| 0 | 5 | 135.5 ± 18.0 | - | - |
| 128 | 6 | 134.5 ± 20.7^{c} | 0.036 | -1% |
| 256 | 7 | 98.4 ± 23.3^{c} | 1.261 | -27% |
| 512 | 7 | 115.9 ± 20.5^{c} | 0.717 | -14% |
| | | | | |

| Diazepam (mg/kg)^{a} | | | | |
|---|---|---|---|---|
| 4 | 9 | 55.8 ± 20.6^{d} | 2.909 | -59% |
| 8 | 10+^{e} | 16.3 ± 6.4^{f} | 6.222 | -88% |

| | | | | |
|---|---|---|---|---|
| ^{a} Isovaleramide and diazepam were administered i.p. 60 minutes before the rotarod test. | | | | |
| ^{b} Ten rats per group. | | | | |
| ^{c} Not significant according to Student's t Test. | | | | |
| ^{d} p<0.01 according to Student's t Test. | | | | |
| ^{e} p<0.05 according to Fisher's Exact Test. | | | | |
| ^{f} p<0.001 according to Student's t Test. | | | | |

Isovaleramide, administered at doses up to 150 mg/kg (i.p.) 15 minutes before a test on the rotarod in the Frings mouse, did not affect performance significantly. In contrast, doses of 300 mg/kg, 600 mg/kg, and 1000 mg/kg (i.p.) decreased rotarod performance in 1/8, 4/8, and 8/8 of Frings mice tested, respectively.

### Example 2

### Anticonvulsant Activity in the Frings Audiogenic Seizure-Susceptible Mouse Model of Epilepsy

The results of Table 2 demonstrate isovaleramide anticonvulsant activity in this animal model of epilepsy. Isovaleramide also displayed a quick onset and a relatively short duration of action. Anticonvulsant activity was noted as early as 15 minutes, but decreased substantially by two hours. All quantitative studies therefore were conducted at 15 minutes. At this time point, the median effective dose (ED₅₀) for protection against tonic extension was 126 mg/kg, i.p. In addition, a dose-dependent reduction in seizure score was observed at this time point. At doses markedly higher than those providing anticonvulsant activity (>300 mg/kg), animals treated with isovaleramide displayed behavioral toxicity that was characterized by their inability to maintain their balance on the rotarod. No notable toxicity was observed at doses less than 300 mg/kg. The median toxic dose (TD₅₀) for rotarod impairment was 531 mg/kg, i.p. Thus, the calculated protective index (TD₅₀/ED₅₀) was about 4.2.

Therefore, despite the relatively low potency of isovaleramide in this model, it still displayed a relatively good separation between activity and toxicity. Isovaleramide thus had a surprising and unexpected efficacy, based on existing structure-activity relationships for amides and their corresponding acids, as an anticonvulsant in the Frings audiogenic seizure-susceptible mouse model of reflex epilepsy. The activity profile of isovaleramide is similar to that of the broad-spectrum anticonvulsant, sodium valproate. Compounds similar in structure to valproate as well as isovaleric acid have been shown in previous literature to elevate GABA levels throughout the CNS. It is this function, primarily, that the anticonvulsant activity of valproate is attributed to, although other mechanisms have been suggested. Isovaleric acid, on the other hand, was found to be inactive as an anticonvulsant, although it was reported to elicit a slight increase in GABA levels in the brains of mice. For example, see Löscher *et al*., *Neuropharmacology* 24: 427 (1985) ; Keane *et al*., *loc*. *cit*. 22: 875 (1983); Keane *et al*., *Pharmacol. Res. Commun.* 17: 547 (1985).

**Table 2**

| **Effect of Isovaleramide on the Audiogenic Seizure Susceptibility of Frings Mice Following Intraperitoneal Administration** | | | |
|---|---|---|---|
| Dose of Isovaleramide (mg/kg, i.p.) | Seizure Score ± S.E.M. | Number^{a} Protected of Eight Mice Tested | Number^{a} Showing Toxicity of Eight Mice Tested |
| 75 | 4.4 ± 0.6 | 1 | 0 |
| 112.5 | 4.0 ± 0.6 | 2 | 0 |
| 150 | 2.0 ± 0.6 | 6 | 0 |
| 300 | 1.0 ± 0 | 8 | 1 |
| 600 | - | | 4 |
| 1000 | - | | 8 |
| ED₅₀ for protection: 126 mg/kg (98.8 - 168^{b}) | | | |
| TD₅₀: 531 mg/kg (372 - 711^{b}) | | | |

| | | | |
|---|---|---|---|
| ^{a} Measured at 15 minutes. | | | |
| ^{b} 95% confidence interval. | | | |

In general, the historical literature on the structure-activity relationships of anticonvulsant activity around compounds similar to valproate have taught away from simple, unsubstituted compounds such as isovaleramide. It is thus a surprising and unexpected observation that isovaleramide has demonstrated an efficacy profile similar to that of valproate in the Frings audiogenic seizure-susceptible mouse model and a similar separation of activity between efficacy and toxicity as measured by rotarod performance. These observations indicate that isovaleramide is an effective therapeutic agent as a broad-spectrum anticonvulsant. Isovaleramide is known for its relative lack of toxicity in mutagenicity and cytotoxicity tests. See U.S. patent No. 5,506,268 and PCT application WO 94/28,888. On the other hand, valproate has long been noted for its hepatotoxicity-causing profile. For example, see Löscher *et al*., *Neuropharmacology* 24: 427 (1985).

## Claims

1. Use of isovaleramide in the preparation of a pharmaceutical formulation for use in a method of treating a pathology selected from convulsions, epilepsy, headache and spasticity, whereby at least one symptom of said pathology is alleviated.

2. A use according to claim 1, wherein said pathology is convulsions.

3. A use according to claim 1, wherein said pathology is epilepsy.

4. A use according to claim 1, wherein said pathology is headache.

5. A use according to claim 1, wherein said pathology is spasticity.

## Patentansprüche

1. Verwendung von Isovaleramid bei der Herstellung einer pharmazeutischen Formulierung für die Verwendung bei einem Verfahren zur Behandlung eines pathologischen Befundes, der unter Konvulsionen, Epilepsie, Kopfschmerzen und spastischem Zustand ausgewählt ist, wobei wenigstens ein Symptom dieses pathologischen Befundes gelindert wird.

2. Verwendung nach Anspruch 1, bei der der pathologische Befund Konvulsionen sind.

3. Verwendung nach Anspruch 1, bei der der pathologische Befund Epilepsie ist.

4. Verwendung nach Anspruch 1, bei der der pathologische Befund Kopfschmerzen sind.

5. Verwendung nach Anspruch 1, bei der der pathologische Befund ein spastischer Zustand ist.

## Revendications

1. Utilisation d'isovaléramide dans la préparation d'un formulation pharmaceutique destinée à être utilisée dans une méthode de traitement d'une pathologie choisie entre des convulsions, l'épilepsie, des céphalées et la spasticité, au moins un symptôme de la pathologie étant ainsi soulagé.

2. Utilisation suivant la revendication 1, dans laquelle ladite pathologie consiste en convulsions.

3. Utilisation suivant la revendication 1, dans laquelle ladite pathologie est l'épilepsie.

4. Utilisation suivant la revendication 1, dans laquelle ladite pathologie consiste en céphalées.

5. Utilisation suivant la revendication 1, dans laquelle ladite pathologie est la spasticité.
